# EUROPEAN PATENT SPECIFICATION

(11) **EP 1 657 248 B1**
(45) Date of publication and mention of the grant of the patent: **26.10.2011**
(21) Application number: 05017940.7
(22) Date of filing: 18.08.2005
(51) Int. Cl.: C07K 14/285, A61K 39/102, C12N 15/09

(54) **Vaccine for preventing and treating porcine progressive atrophic rhinitis**
Impfstoff zur Prävention und Behandlung von progressiver atrophischer Rhinitis in Schweinen
Vaccin pour la prevention et le traitement de la rhinite atrophique progressive porcine

(30) Priority: 20.08.2004 CN 200410057590
(43) Date of publication of application: 17.05.2006
(73) Proprietor: National Chung-Hsing University, Taichung City (TW)
(72) Inventor: Chien, Maw-Sheng, West District Taichung (TW); Liu, Cheng-I, South District Taichung (TW); Liao, Chih-Ming, Er-Lun Hsiang Yun-Lin County (TW)
(74) Representative: Roberts, Michael Austin

(56) References cited:
- WO-A-94/08011
- US-A- 6 110 470
- PETERSEN S K ET AL: "RECOMBINANT DERIVATIVES OF PASTEURELLA-MULTOCIDA TOXIN CANDIDATES FOR A VACCINE AGAINST PROGRESSIVE ATROPHIC RHINITIS" INFECTION AND IMMUNITY, vol. 59, no. 4, 1991, pages 1387-1393, XP002373280 ISSN: 0019-9567
- NIELSEN J P ET AL: "VACCINATION AGAINST PROGRESSIVE ATROPHIC RHINITIS WITH A RECOMBINANT PASTEURELLA-MULTOCIDA TOXIN DERIVATIVE" CANADIAN JOURNAL OF VETERINARY RESEARCH, vol. 55, no. 2, 1991, pages 128-138, XP002373281 ISSN: 0830-9000
- SAKANO TETSUYA ET AL: "Effect of Bordetella bronchiseptica and serotype D Pasteurella multocida bacterin-toxoid on the occurrence of atrophic rhinitis after experimental infection with B. bronchiseptica and toxigenic type A. P. multocida" JOURNAL OF VETERINARY MEDICAL SCIENCE, vol. 59, no. 1, 1997, pages 55-57, XP002373282 ISSN: 0916-7250
- LIAO ET AL: "Immunogenicity and efficacy of three recombinant subunit Pasteurella multocida toxin vaccines against progressive atrophic rhinitis in pigs" VACCINE, BUTTERWORTH SCIENTIFIC. GUILDFORD, GB, vol. 24, no. 1, 9 January 2006 (2006-01-09), pages 27-35, XP005193744 ISSN: 0264-410X

## Description

### BACKGROUND OF THE INVENTION

### 1.FIELD OF THE INVENTION

This invention is generally in the field of veterinary vaccines, vaccine compositions, and methods of producing the same. Particularly, provided herein are the vaccines for immunizing animal against progressive atrophic rhinitis (PAR), which comprising a combination of three fragments of recombinant subunit *Pasteurella multocida* toxins (rsPMT) each having an amino acid sequence that corresponds to the 2-486, 486-986 or 986-1281 amino acid residues of *Pasteurella multocida* toxin (PMT).

### 2.DESCRIPTION OF RELATED ART

Progressive atrophic rhinitis (PAR) is an important upper respiratory disease in swine. The characteristic lesions include turbinate bone hypoplasia, facial distortion and nasal hemorrhage as a result of frequent sneezing. Moreover, PAR causes significant global economic loss in swine production due to growth retardation. Several studies have demonstrated that *Pasteurella multocida* toxin (PMT) is the major virulence factor responsible for the turbinate atrophy seen in PAR (see, for example, Ackermann MR et al. 1996; Am J Vet Res 57(6):848-852; and Lax AJ & Chanter N. 1990; J Gen Microbiol 136:81-87). Inoculation of PMT alone could reproduce all major symptoms of PAR in experimentally challenged pigs. Either aerosolized or injected into swine, PMT causes severe turbinate atrophy and reduces weight gain (Kamp EM & Kimman TG 1988; Am. J. Vet. Res. 49:1844-1849). The mechanisms by which PMT reduces weight gain and conchal bone atrophy have been widely studied. Results of several studies indicate that PMT could increase bone resorption and reduce bone formation by altering the functions of osteoblasts and osteoclasts.

The molecular basis for the virulence of PMT remains unclear, but may be associated with the activation of osteoclasts or inactivation of osteoblasts. It has been demonstrated that PMT is a potent mitogen for several types of cells such as Swiss 3T3 fibroblasts. PMT was able to induce half-maximal stimulation of DNA synthesis and cell proliferation at doses as low as 1 pM. The effect of PMT on porcine osteoclasts and osteoblasts has been investigated using an *in vitro* cell culture system. Exposure of bone marrow cells to Vitamin D3 and PMT during growth led to an increase in cell numbers and earlier appearance of osteoclasts compared to controls. Low concentrations of PMT resulted in growth retardation and decreased nodule formation in osteoblasts, while high concentrations of PMT increased cell death and inhibited nodule formation (Gwaltney SM et al. 1997; Vet Pathol 34(5):421-430). PMT also stimulates cell proliferation, but impairs cell maturation and cell function in primary cultures of rat osteoblasts. These findings suggest that PMT may increase bone resorption and decrease bone apposition, eventually leading to progressive osteolysis and continuous bone atrophy.

Many potential bacterial pathogens can colonize the nasal cavity or tonsils of swine and *P. multocida* is one of the primary opportunistic pathogens able to cause porcine respiratory disease complex (PRDC). In fact, PAR is considered a contagious respiratory disease with high prevalence throughout the areas of the world where modern pig husbandry is practiced. Antibiotics, vaccination and good management can reduce the severity and frequency of PAR. However, overuse of antibiotics is a source of public health concern and vaccination has emerged as the most attractive approach in controlling PAR (Foged NT et al. Vet Rec 1989; 125(1):7-11; Kobisch M, Pennings A, Vet Rec 1989; 124(3):57-61; and Sakano T et al. J Vet Med Sci 1997; 59(1):55-57).

The entire PMT gene (*toxA*) encoding a protein of 1285 amino acids has been cloned and expressed in *E*. *coli* (Petersen SK & Foged NT 1989; Infect Immun 57(12):3907-3913). A recombinant PMT derivative lacking N-terminal amino acid residues 27-147 was shown to induce a protective response against challenge with a lethal dose of PMT in mice (Petersen SK et al. 1991; Infect Immun 59(4):1387-1393), and to reduce colonization by toxigenic *P. multocida* in the nares and tonsils of swine (Nielsen JP et al. 1991; Can J Vet Res 55(2):128-138). Thus, recombinant PMT derivatives may serve as ideal immunogens to elicit a good protective response without cytotoxicity in animals.

Formalin is the most common reagent used to inactivate PMT, but it may induce chemical alterations that can reduce the immunogenicity or efficacy of vaccines (Nielsen JP *et al*. 1991; as described). Therefore, a non-toxic but immunogenic PMT derivative could be advantageous to the development of effective vaccines against PAR. Most of the PAR vaccines tested to date consist of inactivated cultures of *P. multocida* or PMT toxoids. The toxoids are prepared by treatment of PMT with formaldehyde, which eliminates toxicity while maintaining antigenicity. These PAR vaccines are effective when tested on farms. However, PMT constitutes less than 0.6% of the total cellular proteins of *P. multocida* making it necessary to culture a large quantity of bacteria in order to obtain sufficient antigen for commercial scale use. Traditional toxoid vaccines require large scaled culture of a toxigenic strain of *P. multocida* and a tedious, expensive procedure for preparation of the PMT toxoid. In addition to being both time-consuming and expensive, the need to use inactivating reagents such as formaldehyde may induce uncontrollable chemical alterations in the immunogenicity of proteins that can reduce or eliminate the efficacy of such vaccines.

The N-terminal portion of PMT (residues 1 to 506 a.a.) has been considered to contain the putative cell binding domain and translocation domain. Immunization of the N-terminal rsPMT Tox1 (residues 1 to 487 a.a.) could elicit neutralizing antibodies that could prevent PMT from binding to the target cells and subsequently translocating across the cell membrane. Consequently, the PMT activity was blocked. In addition, the C-terminal portion of PMT was suggested as the catalytic domain and antibodies raised against C-terminal fragments (residues 681-1285 and 849-1285) were capable of inhibiting the mitogenic effect of PMT. The residues 1165 (cysteine), 1205 (histidine) and 1223 (histidine) were showed to be essential for the intracellular activity of PMT (Baldwin MR *et al*. 2004; 54(1):239-250; and Pullinger GD et al. 2001; Infect. Immun. 69: 7839-7850). The rsPMT derivatives are easy to produce and not cytotoxic so that no extra chemical inactivation is required before use. US patent no. 6,110,470 disclosed polypeptide derivatives of *P. multocida* toxin comprising amino acid sequences identical to PMT but lacking amino acids 1043-1130 or lacking amino acids 1130-1285.

Therefore, in this invention, three recombinant subunit PMT (rsPMT) derivatives, representing the N-terminal (aa. 2-486), middle (aa. 486-986), and C-terminal (aa. 986-1281) portions of PMT, are produced and their immunogenicities are characterized by assessing the level of PMT-specific antibody secreting cells, the serum neutralizing antibody titers and the degree of lymphocyte proliferation in immunized mice and swine. The efficacy of these recombinant subunits as a vaccine was also evaluated in pregnant sows and their offspring by analysis of neutralizing antibody titers in colostrum and serum, and by monitoring the survival rate and the mean weight gain in piglets after PMT challenge.

### SUMMARY OF THE INVENTION

One aspect of the present invention is to provide a vaccine for immunizing animal against progressive atrophic rhinitis (PAR), which comprises a combination of three proteins each comprising only one fragment of *Pasteurella multocida* toxins (rsPMT) wherein the fragment consists of the amino acid sequence of the 2-486, 486-986 or 986-1281 amino acid residues in SEQ ID No: 2 respectively.

In one embodiment, the fragments of PMT are manufactured by host cell that has been transformed with a plasmid comprising the coding sequence of *Pasteurella multocida* toxin fragment 2-486, 486-986 and/or 986-1281. The host cell used herein may be prokaryotic or eukaryotic.

In the second aspect, the present invention provides a multivalent vaccine against progressive atrophic rhinitis (PAR), which comprises a combination of three proteins as described above as the first component; and at least one antigen or epitope associated with another animal pathology. In one embodiment, the multivalent vaccine is used for preventing and/or treating progressive atrophic rhinitis (PAR) and pseudorabies (PR).

### BRIEF DESCRIPTION OF THE FIGURE

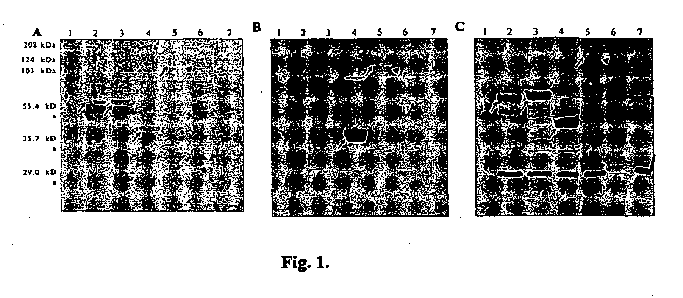

Figure 1 depicts the total cellular proteins expressed by different rsPMT clones in *E. coli* were separated on 10% SDS-PAGE (A), followed by the Western blotting analysis using anti-PMT monoclonal antibody (B), or swine immune serum (C). Lane 1, protein standards (BIO-RAD); lane 2, Tox1 (86 kDa); lane 3, Tox2 (86 kDa); lane 4, Tox7 (55.4 kDa); lane 5, Tox6 (158 kDa); lane 6, PMT (155 kDa); lane 7, pET 32b(+). The location of each expressed rsPMT protein is indicated by an arrow, and native PMT synthesized in *P. multocida* PMD 48 is indicated by an arrowhead.

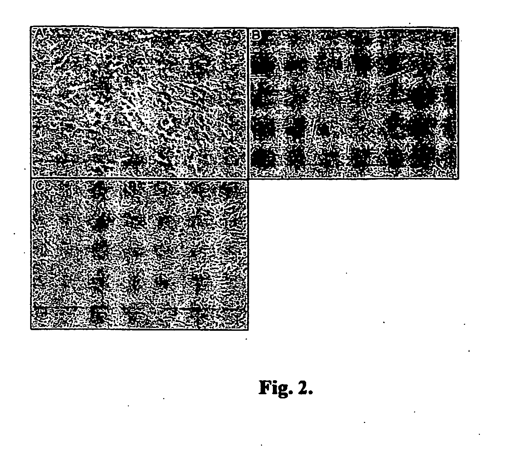

Figure 2 depicts the cytotoxicity of rsPMT and native PMT on Vero cells. Monolayers of Vero cells were treated with DMEM (A), 140 ng/ml native PMT (B), and 1.5 mg/ml Tox1 (C), respectively at 37°C for 7 days. The cellular morphology is visualized by the phase-contrast microscope (Olympus IX-70). Magnification 100X.

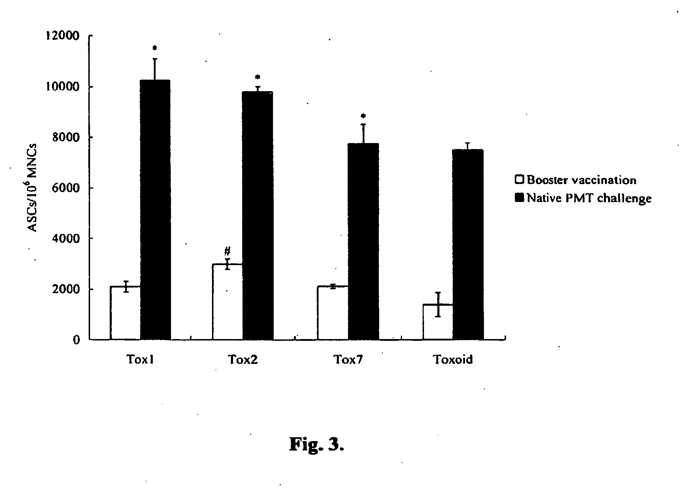

Figure 3 depicts the PMT-specific ASCs of immunized mice present in spleen at 14 days post booster vaccination (open bars) and lethal dose of native PMT challenged (closed bars). At each time point, 3 mice/group were studied. ^{#}Significantly (*p*<0.05) different with toxoid-immunized mice after booster vaccination. *Significantly (*p*<0.05) different with toxoid-immunized mice after native PMT challenged.

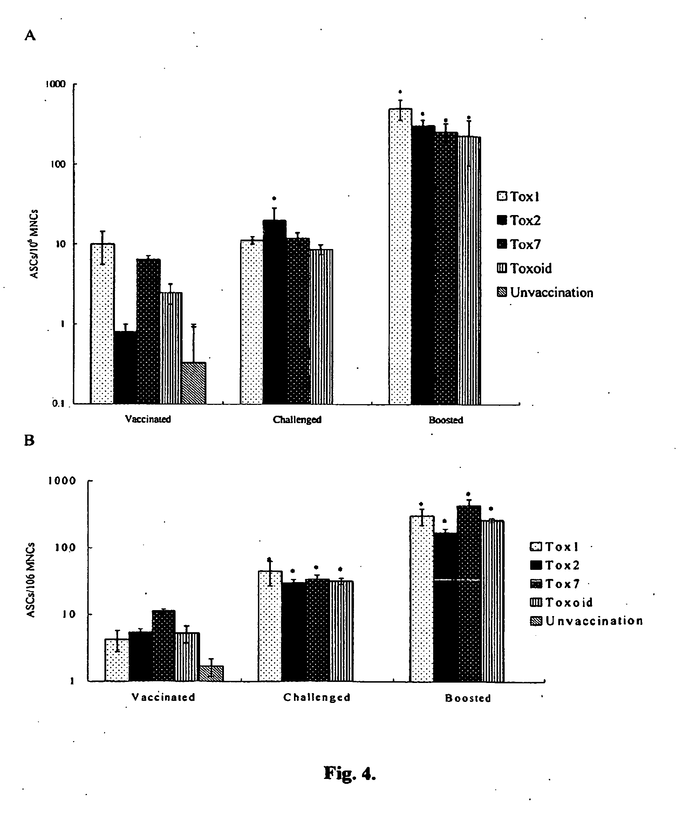

Figure 4 depicts PMT-specific ASCs detected in pulmonary lymph node (A) and spleen (B) of piglets after booster immunization (vaccinated), and subsequent PMT challenge (challenged) or homologous antigen booster (boosted). The results of each experiment were analyzed for effect of treatment using Student's *t* distribution. Statistical results were considered to be significant when *p*-values were lower than or equal to 0.05 (*).

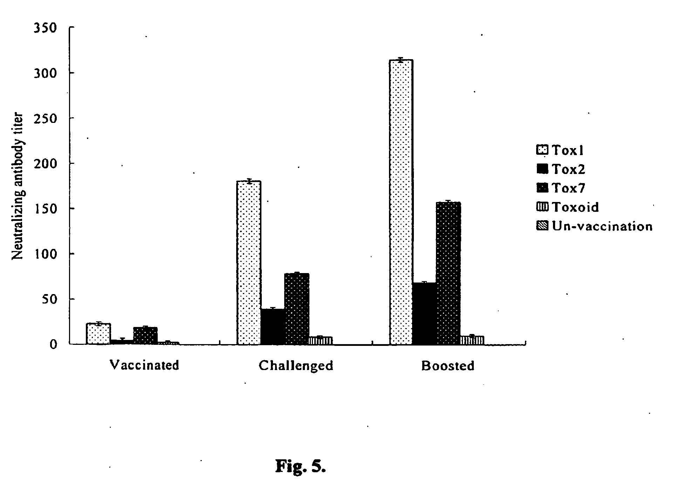

Figure 5 depicts PMT-specific serum neutralizing antibody titers in piglets after boost immunization (vaccinated), and subsequent PMT challenging (challenged) or homologous antigen booster (boosted). The SN titer was expressed as the end-point dilution of serum that could inhibit the cytotoxicity of 4-fold MTD of authentic PMT on Vero cells.

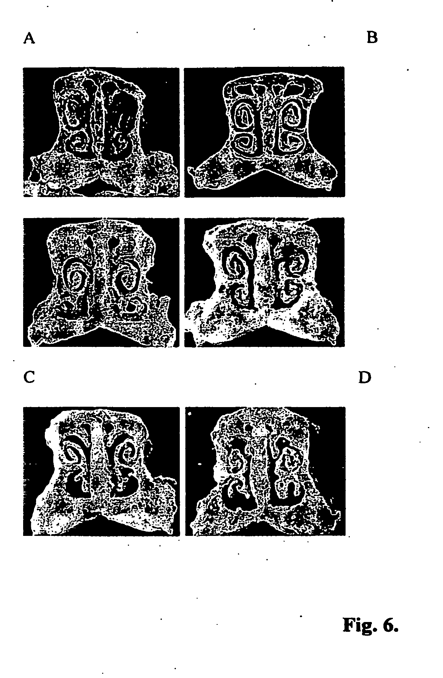

Figure 6 depicts the representative photographs of the turbinate conchae of experimental pigs in groups vaccinated with rsPMTs vaccine (B), conventional AR-toxoid vaccine (C), and unvaccinated (D), at 2-weeks after authentic PMT challenge. The unvaccinated and unchallenged pigs were served as the negative control (A).

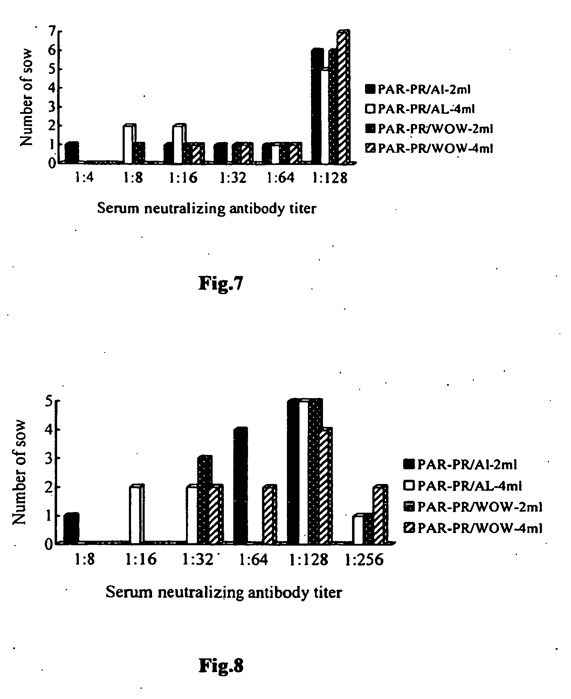

Figure 7 depicts the PMT-specific serum neutralizing antibody titers in pregnant saws before and after boost immunization (vaccinated) of the PAR-PR bivalent vaccine.
Figure 8 depicts the PR-specific serum neutralizing antibody titers in pregnant sows before and after boost immunization of the PAR-PR bivalent vaccine.

### DETAILED DESCRIPTION

Unless defined otherwise, all technical and scientific terms used herein have the same meaning as is commonly understood by one of skill in the art of the invention.

The vaccine according to the present invention comprises a combination of three fragments of recombinant subunit *Pasteurella multocida* toxins (rsPMT) each having an amino acid sequence that consists of the 2-486, 486-986 or 986-1281 amino acid residues in SEQ ID No: 2. The fragments of rsPMT may be expressed in prokaryotic or eukaryotic host cell transformed with a plasmid comprising the coding sequence of the *Pasteurella multocida* toxin fragments.

As used herein, a combination refers to any association between or among two or more elements.

As used herein, production by recombinant DNA technique by using recombinant DNA methods means the use of the well-known methods of molecular biology for expressing proteins encoded by cloned DNA.

As used herein, the term "vector" refers to a nucleic acid molecule capable of transporting another nucleic acid to which it has been linked. In general, expression vectors are in the form of "plasmid", which are generally circular double stranded DNA loops that are not bound to the chromosome.

As used herein, "amino acid residue" refers to an amino acid formed upon chemical digestion (hydrolysis) of a polypeptide at its peptide linkages.

The amino acid sequence that "substantially corresponds to" means with homology of about greater than or equal to 80%, 85%, 90%, 95% or 99% sequence homology; the precise percentage can be specified if necessary.

Examples of conventional adjuvant used in the present vaccine formulations include Aluminum compounds, also known as aluminum gel, such as aluminum hydroxide, Al(OH)₃ and aluminum phosphate, AlPO₄; potassium aluminum sulfate, KAl(SO₄)₂·12H₂o (D.E.S. Stewart-Tull (1996), Aluminum adjuvants. In Vaccine protocols, Robinson, A., G. H., and C. N. Wiblin, Farrar Human Press. Totoga, New Jersey, USA. pp. 135-139); Freund's complete adjuvant, FCA; Freund's incomplete adjuvant, FIA; water-in-oil, W/O emulsion; oil-in-water, O/W emulsion and the likeo

Concanavalin A, Con A is an effective immunostimulant which activating T cells. The proliferative responses of T lymphocytes secrete IL-2 and other cytokines for promoting the associated immune responses.

### Examples

Other features of the invention will become apparent in the course of the following descriptions of exemplary embodiments. These examples are given for illustration of the invention and are not intended to be limiting thereof.

### Example 1. Construction of derivative clones of PMT

*Pasteurella multocida* PMD 48 is a type D toxigenic isolate obtained from a pig affected by a typical case of PAR in Taiwan (Liao CM et al. 2002; Taiwan Vet J 28(4):281-293.). *P. multocida* PMD 48 was cultured in Brain Heart Infusion (BHI) broth (Difco) for authentic *Pasteurella multocida* toxin preparation and genomic DNA extraction. The *E. coli* BL21 (DE3) strain (Novagen) was cultured in Luria-Bertain (LB) medium for cloning and protein expression. The PMT protein coding sequences were cloned into the T7 promoter-based pET expression vectors (Novagen). Restriction enzymes and T4 DNA ligase were purchased from New England Biolabs.

A full-length PMT gene product was created by polymerase chain reaction (PCR) using PMT-specific primers (forward:
5'AGAGGTTATGGATCCGAAAACAAAACATTTT3'; reverse: 5'CTCTTGTTAAGCTAGCCTTTGTGAAAAGAGGAG3'). The full-length gene product was purified then digested with appropriate combinations of restriction enzymes to produce three different coding regions of the PMT gene. The 1459 bp *BamH*I/*Hind*III fragment encoding N-terminal amino acids 1-487 of PMT was cloned into pET32b to generate the Tox1 clone.

The 1508 bp *Hind*III /*Hind*III fragment encoding the middle region (aa 485 to 987) of PMT was cloned into pET32a to generate the Tox2 clone. The 891 bp *Hind*III*lNhe*I fragment encoding the C-terminal region (aa 986 to 1282) of PMT was cloned into pET25b and followed by subcloning the *Bam*HI/*Blp*I fragment into pET32b to generate the C-terminal Tox7 clone.

Recombinant expression plasmids Tox1, Tox2, and Tox7 were transformed into *E. coli* BL21 (DE3) according to the manufacturer's manual. The rsPMT expression was induced with 1 mM

Isopropyl-β-D-thiogalactopyranoside (IPTG; Protech), and rsPMT was purified using the His Bind^{®} Kits (Novagen, Darmstadt, Germany) according to the manufacturer's manual. The protein concentration was quantified using the Bio-Rad Protein Assay reagent (BIO-RAD, Hercules,

CA). Authentic PMT was prepared from *P. multocida* PMD 48 cultured in BHI medium at 37 °C for 26 h as previously described (Nakai T et al. 1984; Infect Immun 46(2):429-434). The authentic PMT was detoxified with 0.3% (v/v) formalin (Fisher) at 37 °C with shaking for 48 h to generate PMT toxoid.

### Example 2. Expression and purification of rsPMT

The rsPMTs were expressed as fusion proteins containing an N-terminal fusion peptide. Plasmids Tox1, Tox2, Tox6 and Tox7 were transformed into competent *E. coli* BL21 (DE3) cells according to the manufacturer's instructions. A single colony of each transformant was grown at 37 °C in Luria-Bertain (LB) medium containing 100 µg/ml ampicillin until the OD₆₀₀ reached 1.0. Isopropyl-β-D-thiogalactopyranoside (IPTG) was then added to a final concentration of mM. The culture was incubated for an additional 6 hr at 37 °C. The cells were harvested by centrifugation and resuspended in phosphate buffered saline (PBS) with 0.1% Triton X-100. Cells were broken by sonication and the suspension were mixed with an equal volume of 2x SDS-PAGE sample buffer (125 mM Tis-HCl [pH6.8], 20% glycerol, 4% SDS, 10% β-mercaptoethanol, 0.25% bromophenol blue) and the proteins were separated by SDS-PAGE. Native PMT was prepared from *P*. *multocida* PMD 48 cultured in BHI medium at 37 °C for 26 hr as previously described (21). After cells were broken, the insoluble fractions containing rsPMTs were harvest by the centrifugation. The insoluble fractions were dissolved in solubilization buffer (50 mM CAPS, 0.3% N-lauroylsarcosine, 1 mM DTT; Novagen, Darmstadt, Germany) and incubated at room temperature for 15 min. After centrifugation, supernatant containing the solubilized protein was transferred to a clean tube for further recombinant protein purification. The rsPMT was purified using the His Bind^{®} Kits (Novagen, Darmstadt, Germany) according to the manufacturer's manual, followed by refolding in 10-fold volumes of PBS at 4 °C overnight. After concentrated with Amicon^{®} Ultra 30,000 MWCO (Millipore, Bedford, USA), the protein concentration was quantified using the Bio-Rad Protein Assay reagent (BIO-RAD, Hercules, CA).

Three recombinant subunit PMT proteins representing the N-terminal (Tox1; aa 1 to 487), the middle (Tox2; aa 485 to 987), and C-terminal (Tox7; aa 986 to 1282) regions of PMT, respectively, were successfully produced in *E. coli*. The molecular weight of Tox1, Tox2, and Tox7 on 10% SDS-PAGE was 86, 86, and 55.4 kDa, respectively (Fig. 2). The expression efficiencies of rsPMT proteins ranged from 28-35% of the total cellular protein (data not shown). The expression of rsPMT was remarkably increased up to 60 fold in the total cellular proteins. These results suggest that, as compared with production of native PMT, sufficient quantities of rsPMT proteins could be obtained to significantly decrease the costs of vaccine preparation.

### Example 3. Cytotoxicity assay of rsPMT in mice

African green monkey kidney cells (Vero, ATCC CCL-81) were obtained from Food Industry Research and Development of Taiwan, R.O.C. and cultured in DMEM supplemented with 2 mM L-glutamine, 1.5 g/l sodium bicarbonate, 0.1 mM sodium pyruvate and 5% fetal calf serum (FCS, Gibco/BRL). Vero cells were seeded into the wells of 96-well plates (Costar) at a density of 5×10⁴ cells per well and the plates were incubated at 37 C overnight. Serial dilutions of rsPMT or native PMT proteins were added to the cell monolayers and the cells were incubated in DMEM containing 2% FCS at 37 °C for 5-7 days. Cytopathic effects consisting of nodular formation in the monolayer were visualized by phase-contrast microscope (Olympus IX-70) and the minimal toxic dose (MTD) was calculated for each rsPMT and the native PMT. All of the rsPMT were non-cytotoxic (Fig. 3), even at dosages as high as 1.5 mg/ml. In contrast, the minimal toxic dose (MTD) of native PMT was 140 ng/ml, which was at least 10000-fold more toxic to Vero cells than any of the rsPMT proteins.

*LD₅₀ in mice*. Fifty SPF BALB/c mice were randomly grouped and each mouse was inoculated via intraperitonal (i.p.) injection with 0.5 ml of a suspension containing a selected concentration of rsPMT or native PMT at six-week-old. These mice were observed for 14 days after inoculation and mortality was recorded. The LD₅₀ was determined by the 50% end-point method of Behrens-Karber (13). Mice inoculated with native PMT demonstrated rough hair coat, anorexia and reluctance to move. These animals huddled in the corners of cages and died within 2-3 days. Lesions at necropsy included congestion or hyperemia of organs and atrophy of spleen. The LD₅₀ of native PMT in BALB/c mouse was 1.30 µg. In contrast, no significant clinical symptoms, gross lesions or other pathological findings were observed in mice receiving doses as high as 1 mg of rsPMT proteins (Table 1)

**Table 1. The expression efficiency of native and recombinant subunit PMT, and their 50% lethal dose (LD₅₀) in BALB/c mouse**

| | Recombinant subunit PMT | | | | Native |
|---|---|---|---|---|---|
| | Tox1 | Tox2 | Tox6 | Tox7 | PMT |
| Recombinant protein/ total cellular protein (%)*^{a}* | 28.2±5.07 | 35.8±6.88 | 4.1±0.41 | 32.1±6.92 | 0.6±0.15 |
| LD₅₀ (µg/0.5ml)*^{b}* | >1000 | >1000 | ND | >1000 | 1.30 |

| | | | | | |
|---|---|---|---|---|---|
| *^{a}*The expression efficacy was analyzed with AlphaImager^{™} 2200 image system. *^{b}*LD₅₀ was presented as the protein concentration needed to kill 50% of mice. | | | | | |

### Example 4. Immunoprotective properties of the rsPMT proteins in mice ELISPOT of antibody secreting cells,

A suspension of mouse spleen MNCs was assayed for PMT-specific ASCs by the enzyme-linked immunospot (ELISPOT) assay. The 96-well nitrocellulose bottomed plates (Millititer HA, Millipore Corp) were coated with rsPMT (100 ng/ml) and incubated at 4 C overnight. The plate was washed with PBS containing 0.05% Tween-20 (PBS-T) and blocked with PBS containing 0.5% bovine serum albumin. Following incubation at 37 C for 1 hr, the plate was washed once with PBS-T and incubated with serial dilutions of a suspension of MNCs. Cells were incubated at 37 C in an atmosphere containing 5% CO₂ for a further 6 hr. The plate was washed once with PBS-T, followed by reacting with PBS-diluted alkaline phosphatase-conjugated goat anti-mouse IgG at 37 C for 1 hr. Finally, the plate was washed six times with PBS-T, and reacted with a chromogen/substrate solution NBT/BCIP (Sigma) at room temperature for 15 to 20 min. After rinsing with deionized water, the color spots present on each well were visualized and quantified with stereomicroscope.

The mouse spleens were markedly enlarged following the second immunization. Two weeks following challenge with native PMT, the spleens from Tox1- and Tox2- immunized mice began to atrophy, but all of the mice survived. The greatest numbers of ASCs were detected in the Tox2-immunized mice possessing 2993.33±200.33 ASCs specific to PMT in 10⁶ MNCs. The lowest amount of ASCs (1386.67±477.21 in 10⁶ MNCs) was detected in the PMT toxoid-immunized mice. Except Tox2-, there was no significant difference among the Tox1-, Tox7- and toxoid-immunized mice (*p*<0.05). The ASCs of immunized mice increased significantly in every group after challenge with native PMT (*p*≤<0.001) (Fig. 3). The Tox1-immunized mice demonstrated the greatest increase in the number of ASCs with 10233.33±850.49 in 10⁶ MNCs (Fig. 3).

### Cellular response of immunized mice

Anti-PMT cellular immune response of mice was analyzed by a lymphocyte proliferation assay. The mean stimulation indices of Tox1-, Tox2-, Tox7-, and toxoid-vaccinated mice were 2.11±0.27, 3.31±0.95, 2.31±0.26, and 6.02±0.68, respectively. After challenge with native PMT, only the cells isolated from Tox7- and toxoid-immunized mice could be stimulated with native PMT *in vitro.* The results implied that mice vaccinated with rsPMT proteins could mount a specific cellular response against PMT (*SI*>2), but the response might be inhibited by exposure to native PMT (Table 2).

**Table 2. Lymphocyte proliferation assay of immunized mice at 14 days post booster vaccination and lethal dose of native PMT challenge**

| Grouping | Booster vaccination (n= 3) | PMT Challenge (n=3) |
|---|---|---|
| Mock | 0.99±0.26 | 1.66±0.49 |
| Tox1 | 2.11±0.27 | 1.06±0.49 |
| Tox2 | 3.31±0.95 | 0.96±0.19 |
| Tox7 | 2.31±0.26 | 2.8±0.37 |
| Toxoid | 6.02±0.68 | 8.07±4.13 |

Data were expressed as the stimulation index (*SI*).

*SI*= cpm in antigen stimulated cultures/cpm in unstimulated cultures. An *SI* value greater than 2 was regarded as positive (van Diemen *et al*., 1994)

### Example 5. Protection efficacy of rsPMT vaccine in pigs Antibody responses in immunized piglets

The antigenicity of the rsPMT products was tested in 4-week-old piglets by analyzing the level of PMT-specific antibody secreting cells and titers of neutralizing antibodies after immunization, PMT challenge, or homologous antigen booster. The spleen and pulmonary lymph node antibody secreting cells were quantified by ELISPOT assay. Each staining spot represented one PMT-specific ASC, and total color spots were quantified. Only a few PMT-specific ASCs were detected at 14 days after booster vaccination in each vaccinated group. The greatest number of PMT-specific ASCs in the spleen was detected in Tox7-immunized piglets that had 11.5±0.7 ASCs per 10⁶ MNCs (see, Fig. 4B), and the greatest number of PMT-specific ASCs in lymph nodes was shown in the Tox1 group (Fig. 4A). The amounts of ASCs increased slightly at 4 weeks after authentic PMT challenge, but increased dramatically in the group booster with homologous antigen. The Tox1-immunized piglets demonstrated the greatest increase in ASCs with 493.3±138.7 per 10⁶ MNCs in pulmonary lymph node and Tox7-immunized piglets possessed the greatest increase in ASCs with 440±104.4 per 10⁶ MNCs in spleen MNCs after antigen booster (showed in Fig. 4).

Furthermore, the PMT-specific neutralizing antibody titer was determined as its ability to inhibit the cytopathic effects induced by PMT in Vero cells. After booster vaccination, moderate levels of neutralizing antibody titer (≥1:16) were detected in Tox1 and Tox7 immunized pigs, and a low level of neutralizing antibody titer (1:4) was detected in Tox2 group. The neutralizing antibody titers increased significantly after PMT challenge or booster with homologous rsPMT antigen, but not in the PMT toxoid vaccinated group. The Tox1 immunized pigs could generate the highest neutralizing antibody titers in every assay point and the Tox7 immunized pigs were the next. There was no detectable neutralizing antibody in unvaccinated pigs. In summary, after PMT challenge or homologous antigen booster, the neutralizing antibody titers in recombinant subunit PMT immunized pigs could reach 1:32 to 1:512, but only 1:8 in PMT toxoid immunized pigs (Fig. 5).

### Cellular immune response in immunized piglets

The specific cellular immune response to each recombinant subunit PMT was analyzed by the lymphocyte proliferation assay. Lymphocyte proliferation was measured and presented as the stimulation index (*SI*). After booster vaccination, a PMT-specific lymphocytes proliferation in spleen was observed in Tox2 and Tox7 vaccinated pigs as indicated with a *SI* greater than 2, while no response was detected in pulmonary lymph node. After PMT challenge or homologous antigen booster, the spleen MNCs from each rsPMT or toxoid immunized group all demonstrated significant enhancement of lymphocyte proliferation (Table 3). Except for Tox1-immunized pigs, the pulmonary lymph node MNCs in each group also showed specific proliferation response after homologous antigen booster.

**Table 3. PMT-specific proliferation of lymphocytes in MNCs from pulmonary lymph nodes (PLN) and spleen isolated from immunized piglets after booster vaccination, challenged with PMT, and boosted with homologous antigen**

| Group (Vaccination antigen) | Vaccination | | PMT challenge | | Homologous antigen booster | |
|---|---|---|---|---|---|---|
| | PLN | Spleen | PLN | Spleen | PLN | Spleen |
| Tox1 | 1.1 | 1.0 | 1.2 | 5.6 | 1.8 | 4.8 |
| Tox2 | 1.2 | 4.0 | 1.9 | 4.9 | 3.1 | 5.1 |
| Tox7 | 1.4 | 3.4 | 1.4 | 3.5 | 4.2 | 4.6 |
| Toxoid | 1.5 | 1.8 | 1.0 | 6.9 | 4.0 | 5.6 |

Data are average of the stimulation index (*SI*) of PMT-specific lymphocyte proliferation form three pigs at each time point. The *SI* was calculated as described in Materials and Methods, and *SI*>2 represent lymphocyte proliferation.

### Protection efficacy of rsPMT vaccine in piglet

To evaluate the protection efficacy of these rsPMTs in newborn piglets, immunization of the pregnant sows with rsPMTs mixture with or without *P*. *multocida* type A bacterin and a conventional PAR-toxoid vaccine were applied for comparison. The neutralizing antibody titers in sows' colostrum were assayed at parturition and the maternal neutralizing antibody titers in sera from offspring were analyzed at one-day of age. Pregnant sows vaccinated with rsPMTs then given (group A) or not given (group B) an injection of *P. multocida* type A bacterin could mount a significant response with high neutralizing antibody titers in colostrum (≥1:80) that could be transferred successfully to newborn piglets (Table 4). Immunized sows in group A demonstrated a higher antibody response than those in group B. By contrast, the conventional PAR-toxoid vaccine (group C) induced a medium level of neutralizing antibody leading to low antibody titers in their offspring (1:8). Only basal levels of neutralizing antibody titer (≤ 1:4) were detected in the control animals (group D). In addition, ten offspring from each group at 14-day-old were challenged by intramuscular injection with 200 µg/kg (5-fold lethal dose) of authentic PMT. The death of piglets was observed as early as 24 h post-inoculation in groups C and D, but not until 4th days post-inoculation in groups A and B. The survival rates of offspring from groups C and D were 0% but reached 60% in groups A and B at 28-days of age (Table 4).

**Table 4. Neutralizing antibody titers of immunized sows and their offspring, and the survival rate of offspring after challenged with a 5-fold lethal dose (200 µg/kg) of authentic PMT at 14-day-old**

| Group | Vaccine composition | Mean neutralizing antibody titer | | | Survival rate^{a} |
|---|---|---|---|---|---|
| | | Sows | Newborn | Piglets | |
| | | Colostrums | 1-day-old | 28-day-old | |
| A | rsPMTs+*P*. *multocida* bacterin | 1:101 | 1:102 | 1:2 | 60% |
| B | rsPMTs | 1:80 | 1:79 | 1:2 | 60% |
| C | Conventional AR-toxoid vaccine | 1:39 | 1:8 | ND^{b} | 0% |
| D | Unvaccinated | 1 :4 | 1:2 | ND^{b} | 0% |

| | | | | | |
|---|---|---|---|---|---|
| Survival rate was calculated at 28-day-old. ^{b} Not determined, all piglets were dead within 24 h after PMT challenge. | | | | | |

Furthermore, another twenty offspring from each group were tested for weight gain. Half of the piglets in each group were challenged with 30 µg/kg (sublethal dose) of authentic PMT via intramuscular injection at 14-days of age, and the remaining half were untreated. The mean body weight gain of piglets was recorded at 14 days post-inoculation. There was no significant difference among the piglets from three vaccination groups, either challenged or unchallenged, but a significant reduction in weight gain (p<0.05) was observed among piglets in the control group D that were challenged with authentic PMT compared with their unchallenged cohort (Table 5). In the nasal conchal gross examination, there were low levels of turbinate atrophy with scores ranged from 0.1 to 0.3 in the piglets from sows vaccinated with rsPMTs, even after these piglets were challenged with authentic PMT. In contrast, after authentic PMT challenge, piglets from the conventional AR-toxoid vaccinated and unvaccinated sows showed mild to severe turbinate atrophy with average scores of 1.4 and 3.4, respectively (see, Table 5; and Fig. 6), which were significantly different from rsPMTs vaccinated groups (p<0.05).

**Table 5. Mean weight gain and turbinate conchal score of the offspring piglets after 2 weeks challenged with subleathal dose (30 µg/kg) of authentic PMT**

| Sows group | Vaccine composition | Mean weight gain of piglets (mean ±SD) (kg) | | Mean score of turbinate conchal atrophy^{a} | |
|---|---|---|---|---|---|
| | | Unchallenged | Challenged | Unchallenged | Challenged |
| | rsPMTs+*P*. | | | | |
| A | *multocida* bacterin | 3.9 ± 0.8 | 3.5 ± 0.4 | 0.1 | 0.2 |
| B | rsPMTs Conventional | 5.0 ± 0.6 | 4.5 ± 1.3 | 0.2 | 0.3 |
| C | AR-toxoid vaccine | 4.4 ± 1.0 | 3.5 ± 0.9 | 0.2 | 1.4^{#} |
| D | Unvaccinated | 5.2 ± 0.3 | 3.5 ± 0.8* | 0.2 | 3.4^{#} |

| | | | | | |
|---|---|---|---|---|---|
| ^{a} The degrees of turbinate conchal atrophy were ranged from 0 (normal) to 4 (complete atrophy) * The mean daily weight gain in control group differs significantly (*t*-test, *p*<0.05) ^{#} The mean score of turbinate conchal atrophy in control group differs significantly (*t*-test, *p*<0.05) | | | | | |

There was no significant difference in weight-gain between the toxin-challenged and unchallenged subgroups, but piglets from unvaccinated sows showed poorer growth performance after PMT challenge than those unchallenged (p<0.05). In addition, piglets from sows vaccinated with rsPMTs mixture with or without *P. multocida* type A bacterin exhibited low level of turbinate conchal atrophy after challenged with authentic PMT. In contrast, piglets from the conventional PAR-toxoid vaccinated and unvaccinated sows showed significant atrophy of turbinate conchae. These results indicated that an effective vaccination of sows during pregnancy could protect offspring against PAR.

In conclusion, vaccination with the short fragments of recombinant subunit PMT proteins resulted in high levels of neutralizing antibody and a specific cellular immune response against PMT in swine. Immunization of sows with recombinant subunit PMT vaccine during pregnancy is safe and able to elicit levels of neutralizing antibodies in colostrum that could protect their offspring against PMT. These non-toxic recombinant subunit PMT proteins hold great potential as suitable antigens in developing an effective subunit vaccine against PAR.

### Example 6. Preparation and immunogenicity test of the bivalent vaccine (PAR-PR) against progressive atrophic rhinitis and pseudorabies

The PAR vaccine (comprising 2.1 mg PMT recombinant subunit proteins, each 0.7 mg, 1x10⁹ CFU of inactivated *P. multocida* serotype A, and 1x10⁹ CFU of inactivated *P. multocida* serotype D) was mixed with inactivated gE-deleted pseudorabies virus (10⁸ TCID₅₀), and then the sterile oily adjuvant (W/O/W type) or aluminum gel was added to the mixture to form a PAR-PR bivalent vaccine formulation of 2-ml and 4-ml dosage.

### Immunoprotection of PAR-PR bivalentvaccine in pregnant sows

The pregnant sows were immunized by intramuscularly injecting with the PAR-PR bivalent vaccine formulation comprising aluminum gel or oily adjuvant in 2-ml and 4-ml dosage respectively and collected blood samples for the detection of PMT- and PR-specific serum neutralizing antibody titers. As showed in Fig. 7 and 8, the average PMT and PR neutralizing antibody titers observed in sows immunized with the 2-ml dosed aluminum gel containing bivalent vaccine were 88.4- and 90.4-folds respectively, and of 75.2 and 99.2-folds observed in the 4-ml dosage treated sows. Of the observation in oily adjuvant containing bivalent vaccine treated animals, the average PMT and PR neutralizing antibody titers detected were 88.8- and 92.8-folds in 2-ml dosage groups, respectively. In 4-ml dosage groups, the average PMT neutralizing antibody titer detected was 101.2-folds and the RP neutralizing antibody titer was 110.5-folds

## Claims

1. A vaccine for immunizing an animal against progressive atrophic rhinitis (PAR), which comprises a combination of three proteins each comprising only one fragment of *Pasteurella multocida* toxin (rsPMT) wherein the fragment consists of the amino acid sequence of the 2-486, 486-986 and 986-1281 amino acid residues in SEQ ID No: 2, respectively.

2. The vaccine of claim 1, wherein the fragments of PMT are derived from the recombinant subunit *Pasteurella multocida* toxins (rsPMT) of serotype D isolate.

3. The vaccine of claim 1, wherein each fragment of rsPMT is produced by recombinant DNA technology.

4. The vaccine of claim 3, wherein each fragment of rsPMT is expressed in *E. coli* transformant with a plasmid comprising the coding sequence of *Pasteurella multocida* toxin fragment 2-486, 486-986 and/or 986-1281.

5. The vaccine of claim 1, which further comprising at least one antigen derived from PMT toxoid, serotype A *Pasteurella multocida,* serotype D *Pasteurella multocida* or *Bordetella bronchiseptica.*

6. The vaccine of claim 1, which further comprises at least one adjuvant selected from Freund's complete adjuvant, Freund's incomplete adjuvant, aluminum gel, oily adjuvant (W/O/W), water-in-oil (W/O) emulsion, oil-in-water (O/W) emulsion, Con A, β-glucosan, and combination thereof.

7. A multivalent vaccine against progressive atrophic rhinitis (PAR), which comprises a combination of the three proteins of claim 1 as the first component; and at least one antigen or epitope associated with another animal pathology.

8. The multivalent vaccine of claim 7, which comprises inactivated gE-deleted *Pseudorabies* virus (PR) as the second component.

9. The multivalent vaccine of claim 7, which further comprises at least one antigen derived from PMT toxoid, *Pasteurella multocida* serotype A, *Pasteurella multocida* serotype D or *Bordetella bronchiseptica*.

10. The multivalent vaccine of claim 7 which further comprises at least one adjuvant selected from Freund's complete adjuvant, Freund's incomplete adjuvant, aluminum gel, oily adjuvant (W/O/W), water-in-oil (W/O) emulsion, oil-in-water (O/W) emulsion, Con A, β-glucosan, and combination thereof.

11. The multivalent vaccine of claim 7, wherein the fragments of PMT are derived from the recombinant subunit *Pasteurella multocida* toxins (rsPMT) of serotype D isolate.

12. The multivalent vaccine of claim 8, wherein the fragments of PMT and the PR are produced by recombinant DNA technology.

## Patentansprüche

1. Impfstoff zur Immunisierung eines Tieres gegen progressive atrophische Rhinitis (PAR), der eine Kombination von drei Proteinen umfasst, die jeweils nur ein Fragment von *Pasteurella multocida*-Toxin (rsPMT) umfassen, wobei die Fragmente aus den Aminosäuresequenzen der Aminosäurereste 2-486, 486-986 bzw. 986-1281 von SEQ ID NO: 2 bestehen.

2. Impfstoff nach Anspruch 1, wobei die PMT-Fragmente sich von den rekombinanten *Untereinheit-Pasteurella multocida*-Toxinen (rsPMT) des Serotyp-D-Isolats ableiten.

3. Impfstoff nach Anspruch 1, wobei die einzelnen Fragmente von rsPMT durch rekombinante DNA-Technik hergestellt werden.

4. Impfstoff nach Anspruch 3, wobei die einzelnen Fragmente von rsPMT in einer *E. coli*-Transformanten mit einem Plasmid, das die Kodierungssequenz der *Pasteurella multocida*-Toxinfragmente 2-486, 486-986 und/oder 986-1281 umfasst, expxrimiert werden.

5. Impfstoff nach Anspruch 1, der ferner mindestens ein Antigen umfasst, das sich von PMT-Toxoid, Serotyp A-*Pasteurella multocida,* Serotyp D-*Pasteurella multocida* oder *Bordetella bronchiseptica* ableitet.

6. Impfstoff nach Anspruch 1, der ferner mindestens ein Adjuvans umfasst, das aus komplettem Freund-Adjuvans, inkomplettem Freund-Adjuvans, Aluminiumgel, öligem Adjuvans (W/O/W), Wasser-in-Öl-Emulsion (W/O), Öl-in-Wasser-Emulsion (O/W), ConA, β-Glucosan und Kombinationen davon ausgewählt ist.

7. Multivalenter Impfstoff gegen progressive atrophische Rhinitis (PAR), der eine Kombination der drei Proteine von Anspruch 1 als erste Komponente und mindestens ein Antigen oder Epitop, das mit einer anderen Tierpathologie assoziiert ist, umfasst.

8. Multivalenter Impfstoff nach Anspruch 7, der inaktiviertes gEdeletiertes *Pseudorabies-Virus* (PR) als zweite Komponente umfasst.

9. Multivalenter Impfstoff nach Anspruch 7, der ferner mindestens ein Antigen umfasst, das sich von PMT-Toxoid, Serotyp A-*Pasteurella multocida,* Serotyp D*-Pasteurella multocida* oder *Bordetella bronchiseptica* ableitet.

10. Multivalenter Impfstoff nach Anspruch 7, der ferner mindestens ein Adjuvans umfasst, das aus komplettem Freund-Adjuvans, inkomplettem Freund-Adjuvans, Aluminiumgel, öligem Adjuvans (W/O/W), Wasser-in-Öl-Emulsion (W/O), Öl-in-Wasser-Emulsion (O/W), ConA, β-Glucosan und Kombinationen davon ausgewählt ist.

11. Multivalenter Impfstoff nach Anspruch 7, wobei die PMT-Fragmente sich von den rekombinanten *Untereinheit-Pasteurella multocida-Toxinen* (rsPMT) des Serotyp-D-Isolats ableiten.

12. Multivalenter Impfstoff nach Anspruch 8, wobei die einzelnen Fragmente von PMT und das PR durch rekombinante DNA-Technik hergestellt werden.

## Revendications

1. Vaccin destiné à l'immunisation d'un animal contre la rhinite atrophique progressive (RAP), qui comprend une combinaison de trois protéines, ne comprenant chacune qu'un fragment de la toxine de *Pasteurella multocida* (rsPMT), ledit fragment étant constitué des résidus 2 à 486, 486 à 986 et 986 à 1281 de SEQ ID NO : 2, respectivement.

2. Vaccin selon la revendication 1, dans lequel les fragments de la PMT sont dérivés de toxines sous unitaires recombinantes de *Pasteurella multocida* (rsPMT) issues de l'isolat de sérotype D.

3. Vaccin selon la revendication 1, dans lequel chaque fragment de rsPMT est produit par la technologie de l'ADN recombinant.

4. Vaccin selon la revendication 3, dans lequel chaque fragment de rsPMT est exprimé dans un transformant de *E*. *coli* avec un plasmide comprenant la séquence codante du fragment 2 à 486, 486 à 986 et/ou 986 à 1281 de la toxine de *Pasteurella multocida*.

5. Vaccin selon la revendication 1, qui comprend en outre au moins un antigène dérivé de l'anatoxine PMT, *de Pasteurella* multocida du sérotype A, de *Pasteurella multocida* du sérotype D ou de *Bordetella bronchiseptica*.

6. Vaccin selon la revendication 1, qui comprend en outre au moins un adjuvant choisi parmi l'adjuvant complet de Freund, l'adjuvant incomplet de Freund, un gel d'aluminium, un adjuvant huileux (E/H/E), une émulsion eau dans l'huile (E/H), une émulsion huile dans l'eau (H/E), la Con A, le β-glucosane, et des combinaisons de ceux-ci.

7. Vaccin polyvalent dirigé contre la rhinite atrophique progressive (RAP), qui comprend une combinaison des trois protéines selon la revendication 1 en tant que premier composant ; et au moins un antigène ou un épitope associé à une autre pathologie animale.

8. Vaccin polyvalent selon la revendication 7, qui comprend le virus de la pseudorage (PR) gE-délété inactivé en tant que second composant.

9. Vaccin polyvalent selon la revendication 7, qui comprend en outre au moins un antigène dérivé de l'anatoxine PMT, de *Pasteurella multocida* du sérotype A, de *Pasteurella multocida* du sérotype D ou de *Bordetella bronchiseptica.*

10. Vaccin polyvalent selon la revendication 7, qui comprend en outre au moins un adjuvant choisi parmi l'adjuvant complet de Freund, l'adjuvant incomplet de Freund, un gel d'aluminium, un adjuvant huileux (E/H/E), une émulsion eau dans l'huile (E/H), une émulsion huile dans l'eau (H/E), la Con A, le β-glucosane, et des combinaisons de ceux-ci.

11. Vaccin polyvalent selon la revendication 7, dans lequel les fragments de la PMT sont dérivés de toxines sous-unitaires recombinantes de *Pasteurella multocida* (rsPMT) issues de l'isolat de sérotype D.

12. Vaccin polyvalent selon la revendication 8, dans lequel les fragments de PMT et le PR sont produits par la technologie de l'ADM recombinant.


### SEQUENCE LISTING

<110> National Chung-Hsing University
<120> Vaccine for preventing and treating porcine progressive atrophic rhinitis
<130> CP-19850
<160> 13
<170> PatentIn version 3.2
<210> 1
   <211> 4380
   <212> DNA
   <213> *Pasteurella multocida*
<220>
   <221> CDS
   <222> (219)..(4076)
<400> 1
   aacaagggaa aatagctaga ttagacgata tcgataatat cataaataat atttaaaaat 60
   tacgcccctt gacctagagg ggctttttta ttacatcaaa aaaataaacc caaacactgc 120
   gaatgtttgg ggttttattt ataaccaaaa tacattaata tgtttattaa gtaagcatta 180
   tcttacttta ggaataaact aacatagagg ttatggat atg aaa aca aaa cat ttt 236
   Met Lys Thr Lys His Phe
   1 5
   ttt aac tca gat ttt act gta aaa gga aaa agt gcc gat gaa att ttt 284
   Phe Asn Ser Asp Phe Thr Val Lys Gly Lys Ser Ala Asp Glu Ile Phe
   10 15 20
   aga aga ttg tgt act gat cat cct gac aag caa tta aac aat gta aaa 332
   Arg Arg Leu Cys Thr Asp His Pro Asp Lys Gln Leu Asn Asn Val Lys
   25 30 35
   tgg aaa gaa gtt ttt att aat cgt ttt ggt cag atg atg cta gat act 380
   Trp Lys Glu Val Phe Ile Asn Arg Phe Gly Gln Met Met Leu Asp Thr
   40 45 50
   cct aat ccg aga aag att gta gaa aaa att att aat gaa ggg ctt gaa 428
   Pro Asn Pro Arg Lys Ile Val Glu Lys Ile Ile Asn Glu Gly Leu Glu
   55 60 65 70
   aaa caa ggc ctg aaa aat ata gat cct gaa act aca tat ttc aac att 476
   Lys Gln Gly Leu Lys Asn Ile Asp Pro Glu Thr Thr Tyr Phe Asn Ile
   75 80 85
   ttt tca tct tct gac agc tcc gat ggg aac gtt ttt cat tat aac tct 524
   Phe Ser Ser Ser Asp Ser Ser Asp Gly Asn Val Phe His Tyr Asn Ser
   90 95 100
   tta tca gaa tcc tat cga gtt act gat gcc tgc cta atg aat att ttt 572
   Leu Ser Glu Ser Tyr Arg Val Thr Asp Ala Cys Leu Met Asn Ile Phe
   105 110 115
   gtg gag cgt tat ttt gat gat tgg gac ttg cta aat agc tta gcc agt 620
   Val Glu Arg Tyr Phe Asp Asp Trp Asp Leu Leu Asn Ser Leu Ala Ser
   120 125 130
   aat gga ata tat tca gta gga aaa gaa gga gct tat tat cct gat cat 668
   Asn Gly Ile Tyr Ser Val Gly Lys Glu Gly Ala Tyr Tyr Pro Asp His
   135 140 145 150
   gat tat ggt cca gaa tat aac cct gtt tgg gga cca aac gaa caa att 716
   Asp Tyr Gly Pro Glu Tyr Asn Pro Val Trp Gly Pro Asn Glu Gln Ile
   155 160 165
   tac cat tct aga gtg att gca gat atc ctt tat gct cgc tcc gta tgg 764
   Tyr His Ser Arg Val Ile Ala Asp Ile Leu Tyr Ala Arg Ser Val Trp
   170 175 180
   gat gaa ttt aaa aaa tac ttc atg gag tat tgg caa aaa tat gct cag 812
   Asp Glu Phe Lys Lys Tyr Phe Met Glu Tyr Trp Gln Lys Tyr Ala Gln
   185 190 195
   ctt tat acc gaa atg tta tct gat aca ttt ctt gca atg gct att cag 860
   Leu Tyr Thr Glu Met Leu Ser Asp Thr Phe Leu Ala Met Ala Ile Gln
   200 205 210
   caa tat aca cga caa acg ctt act gat gaa ggc ttt ctt atg gtt tgt 908
   Gln Tyr Thr Arg Gln Thr Leu Thr Asp Glu Gly Phe Leu Met Val Cys
   215 220 225 230
   aac aca tat tat ggc aat aag gaa gaa gtt caa ata act cta cta gat 956
   Asn Thr Tyr Tyr Gly Asn Lys Glu Glu Val Gln Ile Thr Leu Leu Asp
   235 240 245
   atc tat gga tac cct tcc act gat ata att tgt ata gag caa aaa ggg 1004
   Ile Tyr Gly Tyr Pro Ser Thr Asp Ile Ile Cys Ile Glu Gln Lys Gly
   250 255 260
   ctt cct act cct aaa gtg ata ctt tac att cct gga gga aca caa cca 1052
   Leu Pro Thr Pro Lys Val Ile Leu Tyr Ile Pro Gly Gly Thr Gln Pro
   265 270 275
   ttt gtt gaa ttt ctt aat aca gat gat ctg aaa caa tgg att gca tgg 1100
   Phe Val Glu Phe Leu Asn Thr Asp Asp Leu Lys Gln Trp Ile Ala Trp
   280 285 290
   cat tta aaa gat aac aaa cat atg gtc cga ttc cgc aaa cat ttc tcg 1148
   His Leu Lys Asp Asn Lys His Met Val Arg Phe Arg Lys His Phe Ser
   295 300 305 310
   cta aaa caa cgt cag gaa gga gaa acg ttt aca ggt ata gat aaa gca 1196
   Leu Lys Gln Arg Gln Glu Gly Glu Thr Phe Thr Gly Ile Asp Lys Ala
   315 320 325
   ctt caa tat att gca gaa gag tcc cct gaa tgg cct gcc aat aaa tac 1244
   Leu Gln Tyr Ile Ala Glu Glu Ser Pro Glu Trp Pro Ala Asn Lys Tyr
   330 335 340
   atc ctt tat aat ccg aca cat tta gaa aca gaa aat tta ttt aac atc 1292
   Ile Leu Tyr Asn Pro Thr His Leu Glu Thr Glu Asn Leu Phe Asn Ile
   345 350 355
   atg atg aag cga aca gaa cag cgg atg ctt gaa gat agt gat gta cag 1340
   Met Met Lys Arg Thr Glu Gln Arg Met Leu Glu Asp Ser Asp Val Gln
   360 365 370
   att aga tca aat tca gaa gct acc cgt gac tat gct ctt tca tta ctc 1388
   Ile Arg Ser Asn Ser Glu Ala Thr Arg Asp Tyr Ala Leu Ser Leu Leu
   375 380 385 390
   gaa acc ttt att tca cag tta tct gca ata gat atg tta gta cca gca 1436
   Glu Thr Phe Ile Ser Gln Leu Ser Ala Ile Asp Met Leu Val Pro Ala
   395 400 405
   gta ggt atc cca att aat ttt gcc cta tca gct aca gca tta gga ctt 1484
   Val Gly Ile Pro Ile Asn Phe Ala Leu Ser Ala Thr Ala Leu Gly Leu
   410 415 420
   agc tcg gat att gta gtt aat gga gat tca tat gaa aag aga aaa tat 1532
   Ser Ser Asp Ile Val Val Asn Gly Asp Ser Tyr Glu Lys Arg Lys Tyr
   425 430 435
   gga att ggg tcc tta gtg caa tct gca tta ttc aca gga att aat ctt 1580
   Gly Ile Gly Ser Leu Val Gln Ser Ala Leu Phe Thr Gly Ile Asn Leu
   440 445 450
   att cca gtt att tcg gaa acc gca gaa att tta tct tct ttc tct aga 1628
   Ile Pro Val Ile Ser Glu Thr Ala Glu Ile Leu Ser Ser Phe Ser Arg
   455 460 465 470
   aca gaa gaa gat att cca gct ttt ttc act gaa gaa caa gct tta gct 1676
   Thr Glu Glu Asp Ile Pro Ala Phe Phe Thr Glu Glu Gln Ala Leu Ala
   475 480 485
   caa cgc ttt gaa ata gta gaa gaa gaa tta cat tct atc tca cct gat 1724
   Gln Arg Phe Glu Ile Val Glu Glu Glu Leu His Ser Ile Ser Pro Asp
   490 495 500
   gat cct cct cga gaa att act gac gaa aat tta cat aaa att cgt ctg 1772
   Asp Pro Pro Arg Glu Ile Thr Asp Glu Asn Leu His Lys Ile Arg Leu
   505 510 515
   gta cgt ctt aac aat gaa aat caa cct tta gtt gtg tta cga aga tta 1820
   Val Arg Leu Asn Asn Glu Asn Gln Pro Leu Val Val Leu Arg Arg Leu
   520 525 530
   gga gga aat aaa ttt atc aga atc gag cct ata aca ttc cag gaa ata 1868
   Gly Gly Asn Lys Phe Ile Arg Ile Glu Pro Ile Thr Phe Gln Glu Ile
   535 540 545 550
   aaa ggt tct tta gta agt gaa gtt ata aat cca gtg act aat aaa acg 1916
   Lys Gly Ser Leu Val Ser Glu Val Ile Asn Pro Val Thr Asn Lys Thr
   555 560 565
   tac tac gta agc aat gct aaa cta tta ggg ggc tct cct tat agt cct 1964
   Tyr Tyr Val Ser Asn Ala Lys Leu Leu Gly Gly Ser Pro Tyr Ser Pro
   570 575 580
   ttc cgt att gga tta gaa ggt gtt tgg aca cca gag gta tta aaa gca 2012
   Phe Arg Ile Gly Leu Glu Gly Val Trp Thr Pro Glu Val Leu Lys Ala
   585 590 595
   aga gct tcc gtt att gga aag cct att gga gaa tca tat aaa aga ata 2060
   Arg Ala Ser Val Ile Gly Lys Pro Ile Gly Glu Ser Tyr Lys Arg Ile
   600 605 610
   tta gcc aaa cta caa aga ata cat aac agt aat atc tta gat gag cga 2108
   Leu Ala Lys Leu Gln Arg Ile His Asn Ser Asn Ile Leu Asp Glu Arg
   615 620 625 630
   caa ggt tta atg cat gaa ctc atg gag ctt att gat ctt tat gaa gaa 2156
   Gln Gly Leu Met His Glu Leu Met Glu Leu Ile Asp Leu Tyr Glu Glu
   635 640 645
   tcg caa cct tct tca gag cgt ttg aat gct ttt cgt gaa ctg cgt act 2204
   Ser Gln Pro Ser Ser Glu Arg Leu Asn Ala Phe Arg Glu Leu Arg Thr
   650 655 660
   caa tta gaa aaa gcg ctt tat ctt cct gaa atg gaa gca tta aaa aaa 2252
   Gln Leu Glu Lys Ala Leu Tyr Leu Pro Glu Met Glu Ala Leu Lys Lys
   665 670 675
   caa ata cta cag att cct aac aaa ggt tct ggt gcc gct cga ttt tta 2300
   Gln Ile Leu Gln Ile Pro Asn Lys Gly Ser Gly Ala Ala Arg Phe Leu
   680 685 690
   ctt cgt aca gcc atg aat gaa atg gct gga aaa acc agt gaa agc acg 2348
   Leu Arg Thr Ala Met Asn Glu Met Ala Gly Lys Thr Ser Glu Ser Thr
   695 700 705 710
   gct gat tta ata cgc ttt gcc ttg caa gat aca gta att tca gcg cct 2396
   Ala Asp Leu Ile Arg Phe Ala Leu Gln Asp Thr Val Ile Ser Ala Pro
   715 720 725
   ttt cgc gga tat gct ggt gcg att cca gag gca ata gac ttt cct gta 2444
   Phe Arg Gly Tyr Ala Gly Ala Ile Pro Glu Ala Ile Asp Phe Pro Val
   730 735 740
   aaa tat gta ata gaa gac ata tct gta ttt gat aaa ata cag aca aat 2492
   Lys Tyr Val Ile Glu Asp Ile Ser Val Phe Asp Lys Ile Gln Thr Asn
   745 750 755
   tac tgg gaa ctt cct gct tat gaa agc tgg aac gaa gga agt aat agc 2540
   Tyr Trp Glu Leu Pro Ala Tyr Glu Ser Trp Asn Glu Gly Ser Asn Ser
   760 765 770
   cga tta ctg cct ggt ttg tta cgt gaa tcg caa agc aag ggg atg tta 2588
   Arg Leu Leu Pro Gly Leu Leu Arg Glu Ser Gln Ser Lys Gly Met Leu
   775 780 785 790
   agt aag tgt cgt atc ata gaa aat agc ctt tat att gga cat agc tat 2636
   Ser Lys Cys Arg Ile Ile Glu Asn Ser Leu Tyr Ile Gly His Ser Tyr
   795 800 805
   gaa gaa atg ttt tac agc att tct cca tat tca aac cag gtt gga ggg 2684
   Glu Glu Met Phe Tyr Ser Ile Ser Pro Tyr Ser Asn Gln Val Gly Gly
   810 815 820
   cct tat gaa tta tat cct ttc act ttt ttc agt atg ctt caa gaa gta 2732
   Pro Tyr Glu Leu Tyr Pro Phe Thr Phe Phe Ser Met Leu Gln Glu Val
   825 830 835
   caa ggt gat tta gga ttt gag cag gcc ttt gcc aca cgt aac ttt ttc 2780
   Gln Gly Asp Leu Gly Phe Glu Gln Ala Phe Ala Thr Arg Asn Phe Phe
   840 845 850
   aat act ctt gtt tct gat cga cta tcc tta atg gaa aat acg atg tta 2828
   Asn Thr Leu Val Ser Asp Arg Leu Ser Leu Met Glu Asn Thr Met Leu
   855 860 865 870
   ctt aca gaa agt ttt gat tat aca cct tgg gat gct att tat gga gat 2876
   Leu Thr Glu Ser Phe Asp Tyr Thr Pro Trp Asp Ala Ile Tyr Gly Asp
   875 880 885
   att aat tat gat gaa caa ttt gct gca atg tct att aat gaa cgc ata 2924
   Ile Asn Tyr Asp Glu Gln Phe Ala Ala Met Ser Ile Asn Glu Arg Ile
   890 895 900
   gaa aaa tgt atg aat acc tat aga ggt gtg gca ttc caa aac tct tca 2972
   Glu Lys Cys Met Asn Thr Tyr Arg Gly Val Ala Phe Gln Asn Ser Ser
   905 910 915
   aaa agt att gac ttt ttc cta aat aat cta acc aca ttc att gat aat 3020
   Lys Ser Ile Asp Phe Phe Leu Asn Asn Leu Thr Thr Phe Ile Asp Asn
   920 925 930
   gga cta acc gaa att gct ata tct gat tta ccg tat gat att gtg caa 3068
   Gly Leu Thr Glu Ile Ala Ile Ser Asp Leu Pro Tyr Asp Ile Val Gln
   935 940 945 950
   caa gaa atc tct caa ttc tta caa gga agt aat gaa tgg aaa aca ctt 3116
   Gln Glu Ile Ser Gln Phe Leu Gln Gly Ser Asn Glu Trp Lys Thr Leu
   955 960 965
   gat gcc atg tta ttt aac tta gat aaa gga gat att aat ggt gct ttc 3164
   Asp Ala Met Leu Phe Asn Leu Asp Lys Gly Asp Ile Asn Gly Ala Phe
   970 975 980
   aga aag ctt ctg caa tca gca aaa gat aat aat ata aaa ttt aga gct 3212
   Arg Lys Leu Leu Gln Ser Ala Lys Asp Asn Asn Ile Lys Phe Arg Ala
   985 990 995
   ata ggg cat tca gat aat tct gtt ccg cca ttt aat aac cct tat 3257
   Ile Gly His Ser Asp Asn Ser Val Pro Pro Phe Asn Asn Pro Tyr
   1000 1005 1010
   aag tct tta tat tat aaa gga aat ata ata gct gaa gca att gaa 3302
   Lys Ser Leu Tyr Tyr Lys Gly Asn Ile Ile Ala Glu Ala Ile Glu
   1015 1020 1025
   aaa cta gat cga gaa ggt caa aaa ttt gtt gta ttt gct gat agt 3347
   Lys Leu Asp Arg Glu Gly Gln Lys Phe Val Val Phe Ala Asp Ser
   1030 1035 1040
   tct ctg ctc aac agc acg cct ggg aca ggt cgt cct atg cca gga 3392
   Ser Leu Leu Asn Ser Thr Pro Gly Thr Gly Arg Pro Met Pro Gly
   1045 1050 1055
   cta gtt caa tat tta aaa ata cca gca act gta gta gat agc gat 3437
   Leu Val Gln Tyr Leu Lys Ile Pro Ala Thr Val Val Asp Ser Asp
   1060 1065 1070
   ggt gca tgg caa ttt ctt cca gat gta gct tca agc aga gtt cct 3482
   Gly Ala Trp Gln Phe Leu Pro Asp Val Ala Ser Ser Arg Val Pro
   1075 1080 1085
   att gaa gtt aca gag tta gaa aat tgg caa gtc tta act cct cca 3527
   Ile Glu Val Thr Glu Leu Glu Asn Trp Gln Val Leu Thr Pro Pro
   1090 1095 1100
   caa ggt aag att ctt gga tta aag caa ttt aag tta acg gca ggt 3572
   Gln Gly Lys Ile Leu Gly Leu Lys Gln Phe Lys Leu Thr Ala Gly
   1105 1110 1115
   ttt cca aca gaa caa agt cgc tta cct ctt tta gag aat tcg gtt 3617
   Phe Pro Thr Glu Gln Ser Arg Leu Pro Leu Leu Glu Asn Ser Val
   1120 1125 1130
   tct gaa gat tta agg gaa gaa tta atg caa aag att gat gca ata 3662
   Ser Glu Asp Leu Arg Glu Glu Leu Met Gln Lys Ile Asp Ala Ile
   1135 1140 1145
   aaa aat gat gtg aaa atg aat agt tta gtg tgt atg gaa gct ggc 3707
   Lys Asn Asp Val Lys Met Asn Ser Leu Val Cys Met Glu Ala Gly
   1150 1155 1160
   tct tgt gat tca gta agc cct aag gta gct gcc cgt ctt aaa gat 3752
   Ser Cys Asp Ser Val Ser Pro Lys Val Ala Ala Arg Leu Lys Asp
   1165 1170 1175
   atg ggg tta gaa gct ggg atg ggt gct tct att acc tgg tgg aga 3797
   Met Gly Leu Glu Ala Gly Met Gly Ala Ser Ile Thr Trp Trp Arg
   1180 1185 1190
   cgt gaa ggc ggg atg gaa ttt tca cat cag atg cat act act gct 3842
   Arg Glu Gly Gly Met Glu Phe Ser His Gln Met His Thr Thr Ala
   1195 1200 1205
   tcc ttt aaa ttt gct ggt aaa gag ttt gcc gtg gat gct tca cat 3887
   Ser Phe Lys Phe Ala Gly Lys Glu Phe Ala Val Asp Ala Ser His
   1210 1215 1220
   tta caa ttt gta cac gac caa tta gat aca act atc ctg ata cta 3932
   Leu Gln Phe Val His Asp Gln Leu Asp Thr Thr Ile Leu Ile Leu
   1225 1230 1235
   cct gta gat gat tgg gct tta gaa ata gct caa aga aat cgg gct 3977
   Pro Val Asp Asp Trp Ala Leu Glu Ile Ala Gln Arg Asn Arg Ala
   1240 1245 1250
   att aat cct ttt gtg gaa tat gtt agt aaa aca gga aac atg tta 4022
   Ile Asn Pro Phe Val Glu Tyr Val Ser Lys Thr Gly Asn Met Leu
   1255 1260 1265
   gca ctc ttc atg cct cct ctt ttc aca aag cct cgc tta aca aga 4067
   Ala Leu Phe Met Pro Pro Leu Phe Thr Lys Pro Arg Leu Thr Arg
   1270 1275 1280
   gca cta taa ctaattaaaa actgtattaa agccttatat tataaggctt 4116
   Ala Leu
   1285
   taattttctt tcaagaatta ttaagtagaa gaatcaaaat caatgagata gataaaatca 4176
   aatgttatta ccaatacaac tttcttaagt atactttttg aattttttgc gttaataaat 4236
   ttataatacc cttaactcaa taaaagaagt tattgagaag tttaaatctt gtgagcaaga 4296
   tgaagatata atttcagcaa tcgatcttat tagcgcttca tatagaaggg ctgtggatgc 4356
   agtggaacaa agattcggtt ctag 4380
<210> 2
   <211> 1285
   <212> PRT
   <213> *Pasteurella multocida*
<400> 2
   Met Lys Thr Lys His Phe Phe Asn Ser Asp Phe Thr Val Lys Gly Lys
   1 5 10 15
   Ser Ala Asp Glu Ile Phe Arg Arg Leu Cys Thr Asp His Pro Asp Lys
   20 25 30
   Gln Leu Asn Asn Val Lys Trp Lys Glu Val Phe Ile Asn Arg Phe Gly
   35 40 45
   Gln Met Met Leu Asp Thr Pro Asn Pro Arg Lys Ile Val Glu Lys Ile
   50 55 60
   Ile Asn Glu Gly Leu Glu Lys Gln Gly Leu Lys Asn Ile Asp Pro Glu
   65 70 75 80
   Thr Thr Tyr Phe Asn Ile Phe Ser Ser Ser Asp Ser Ser Asp Gly Asn
   85 90 95
   Val Phe His Tyr Asn Ser Leu Ser Glu Ser Tyr Arg Val Thr Asp Ala
   100 105 110
   Cys Leu Met Asn Ile Phe Val Glu Arg Tyr Phe Asp Asp Trp Asp Leu
   115 120 125
   Leu Asn Ser Leu Ala Ser Asn Gly Ile Tyr Ser Val Gly Lys Glu Gly
   130 135 140
   Ala Tyr Tyr Pro Asp His Asp Tyr Gly Pro Glu Tyr Asn Pro Val Trp
   145 150 155 160
   Gly Pro Asn Glu Gln Ile Tyr His Ser Arg Val Ile Ala Asp Ile Leu
   165 170 175
   Tyr Ala Arg Ser Val Trp Asp Glu Phe Lys Lys Tyr Phe Met Glu Tyr
   180 185 190
   Trp Gln Lys Tyr Ala Gln Leu Tyr Thr Glu Met Leu Ser Asp Thr Phe
   195 200 205
   Leu Ala Met Ala Ile Gln Gln Tyr Thr Arg Gln Thr Leu Thr Asp Glu
   210 215 220
   Gly Phe Leu Met Val Cys Asn Thr Tyr Tyr Gly Asn Lys Glu Glu Val
   225 230 235 240
   Gln Ile Thr Leu Leu Asp Ile Tyr Gly Tyr Pro Ser Thr Asp Ile Ile
   245 250 255
   Cys Ile Glu Gln Lys Gly Leu Pro Thr Pro Lys Val Ile Leu Tyr Ile
   260 265 270
   Pro Gly Gly Thr Gln Pro Phe Val Glu Phe Leu Asn Thr Asp Asp Leu
   275 280 285
   Lys Gln Trp Ile Ala Trp His Leu Lys Asp Asn Lys His Met Val Arg
   290 295 300
   Phe Arg Lys His Phe Ser Leu Lys Gln Arg Gln Glu Gly Glu Thr Phe
   305 310 315 320
   Thr Gly Ile Asp Lys Ala Leu Gln Tyr Ile Ala Glu Glu Ser Pro Glu
   325 330 335
   Trp Pro Ala Asn Lys Tyr Ile Leu Tyr Asn Pro Thr His Leu Glu Thr
   340 345 350
   Glu Asn Leu Phe Asn Ile Met Met Lys Arg Thr Glu Gln Arg Met Leu
   355 360 365
   Glu Asp Ser Asp Val Gln Ile Arg Ser Asn Ser Glu Ala Thr Arg Asp
   370 375 380
   Tyr Ala Leu Ser Leu Leu Glu Thr Phe Ile Ser Gln Leu Ser Ala Ile
   385 390 395 400
   Asp Met Leu Val Pro Ala Val Gly Ile Pro Ile Asn Phe Ala Leu Ser
   405 410 415
   Ala Thr Ala Leu Gly Leu Ser Ser Asp Ile Val Val Asn Gly Asp Ser
   420 425 430
   Tyr Glu Lys Arg Lys Tyr Gly Ile Gly Ser Leu Val Gln Ser Ala Leu
   435 440 445
   Phe Thr Gly Ile Asn Leu Ile Pro Val Ile Ser Glu Thr Ala Glu Ile
   450 455 460
   Leu Ser Ser Phe Ser Arg Thr Glu Glu Asp Ile Pro Ala Phe Phe Thr
   465 470 475 480
   Glu Glu Gln Ala Leu Ala Gln Arg Phe Glu Ile Val Glu Glu Glu Leu
   485 490 495
   His Ser Ile Ser Pro Asp Asp Pro Pro Arg Glu Ile Thr Asp Glu Asn
   500 505 510
   Leu His Lys Ile Arg Leu Val Arg Leu Asn Asn Glu Asn Gln Pro Leu
   515 520 525
   Val Val Leu Arg Arg Leu Gly Gly Asn Lys Phe Ile Arg Ile Glu Pro
   530 535 540
   Ile Thr Phe Gln Glu Ile Lys Gly Ser Leu Val Ser Glu Val Ile Asn
   545 550 555 560
   Pro Val Thr Asn Lys Thr Tyr Tyr Val Ser Asn Ala Lys Leu Leu Gly
   565 570 575
   Gly Ser Pro Tyr Ser Pro Phe Arg Ile Gly Leu Glu Gly Val Trp Thr
   580 585 590
   Pro Glu Val Leu Lys Ala Arg Ala Ser Val Ile Gly Lys Pro Ile Gly
   595 600 605
   Glu Ser Tyr Lys Arg Ile Leu Ala Lys Leu Gln Arg Ile His Asn Ser
   610 615 620
   Asn Ile Leu Asp Glu Arg Gln Gly Leu Met His Glu Leu Met Glu Leu
   625 630 635 640
   Ile Asp Leu Tyr Glu Glu Ser Gln Pro Ser Ser Glu Arg Leu Asn Ala
   645 650 655
   Phe Arg Glu Leu Arg Thr Gln Leu Glu Lys Ala Leu Tyr Leu Pro Glu
   660 665 670
   Met Glu Ala Leu Lys Lys Gln Ile Leu Gln Ile Pro Asn Lys Gly Ser
   675 680 685
   Gly Ala Ala Arg Phe Leu Leu Arg Thr Ala Met Asn Glu Met Ala Gly
   690 695 700
   Lys Thr Ser Glu Ser Thr Ala Asp Leu Ile Arg Phe Ala Leu Gln Asp
   705 710 715 720
   Thr Val Ile Ser Ala Pro Phe Arg Gly Tyr Ala Gly Ala Ile Pro Glu
   725 730 735
   Ala Ile Asp Phe Pro Val Lys Tyr Val Ile Glu Asp Ile Ser Val Phe
   740 745 750
   Asp Lys Ile Gln Thr Asn Tyr Trp Glu Leu Pro Ala Tyr Glu Ser Trp
   755 760 765
   Asn Glu Gly Ser Asn Ser Arg Leu Leu Pro Gly Leu Leu Arg Glu Ser
   770 775 780
   Gln Ser Lys Gly Met Leu Ser Lys Cys Arg Ile Ile Glu Asn Ser Leu
   785 790 795 800
   Tyr Ile Gly His Ser Tyr Glu Glu Met Phe Tyr Ser Ile Ser Pro Tyr
   805 810 815
   Ser Asn Gln Val Gly Gly Pro Tyr Glu Leu Tyr Pro Phe Thr Phe Phe
   820 825 830
   Ser Met Leu Gln Glu Val Gln Gly Asp Leu Gly Phe Glu Gln Ala Phe
   835 840 845
   Ala Thr Arg Asn Phe Phe Asn Thr Leu Val Ser Asp Arg Leu Ser Leu
   850 855 860
   Met Glu Asn Thr Met Leu Leu Thr Glu Ser Phe Asp Tyr Thr Pro Trp
   865 870 875 880
   Asp Ala Ile Tyr Gly Asp Ile Asn Tyr Asp Glu Gln Phe Ala Ala Met
   885 890 895
   Ser Ile Asn Glu Arg Ile Glu Lys Cys Met Asn Thr Tyr Arg Gly Val
   900 905 910
   Ala Phe Gln Asn Ser Ser Lys Ser Ile Asp Phe Phe Leu Asn Asn Leu
   915 920 925
   Thr Thr Phe Ile Asp Asn Gly Leu Thr Glu Ile Ala Ile Ser Asp Leu
   930 935 940
   Pro Tyr Asp Ile Val Gln Gln Glu Ile Ser Gln Phe Leu Gln Gly Ser
   945 950 955 960
   Asn Glu Trp Lys Thr Leu Asp Ala Met Leu Phe Asn Leu Asp Lys Gly
   965 970 975
   Asp Ile Asn Gly Ala Phe Arg Lys Leu Leu Gln Ser Ala Lys Asp Asn
   980 985 990
   Asn Ile Lys Phe Arg Ala Ile Gly His Ser Asp Asn Ser Val Pro Pro
   995 1000 1005
   Phe Asn Asn Pro Tyr Lys Ser Leu Tyr Tyr Lys Gly Asn Ile Ile
   1010 1015 1020
   Ala Glu Ala Ile Glu Lys Leu Asp Arg Glu Gly Gln Lys Phe Val
   1025 1030 1035
   Val Phe Ala Asp Ser Ser Leu Leu Asn Ser Thr Pro Gly Thr Gly
   1040 1045 1050
   Arg Pro Met Pro Gly Leu Val Gln Tyr Leu Lys Ile Pro Ala Thr
   1055 1060 1065
   Val Val Asp Ser Asp Gly Ala Trp Gln Phe Leu Pro Asp Val Ala
   1070 1075 1080
   Ser Ser Arg Val Pro Ile Glu Val Thr Glu Leu Glu Asn Trp Gln
   1085 1090 1095
   Val Leu Thr Pro Pro Gln Gly Lys Ile Leu Gly Leu Lys Gln Phe
   1100 1105 1110
   Lys Leu Thr Ala Gly Phe Pro Thr Glu Gln Ser Arg Leu Pro Leu
   1115 1120 1125
   Leu Glu Asn Ser Val Ser Glu Asp Leu Arg Glu Glu Leu Met Gln
   1130 1135 1140
   Lys Ile Asp Ala Ile Lys Asn Asp Val Lys Met Asn Ser Leu Val
   1145 1150 1155
   Cys Met Glu Ala Gly Ser Cys Asp Ser Val Ser Pro Lys Val Ala
   1160 1165 1170
   Ala Arg Leu Lys Asp Met Gly Leu Glu Ala Gly Met Gly Ala Ser
   1175 1180 1185
   Ile Thr Trp Trp Arg Arg Glu Gly Gly Met Glu Phe Ser His Gln
   1190 1195 1200
   Met His Thr Thr Ala Ser Phe Lys Phe Ala Gly Lys Glu Phe Ala
   1205 1210 1215
   Val Asp Ala Ser His Leu Gln Phe Val His Asp Gln Leu Asp Thr
   1220 1225 1230
   Thr Ile Leu Ile Leu Pro Val Asp Asp Trp Ala Leu Glu Ile Ala
   1235 1240 1245
   Gln Arg Asn Arg Ala Ile Asn Pro Phe Val Glu Tyr Val Ser Lys
   1250 1255 1260
   Thr Gly Asn Met Leu Ala Leu Phe Met Pro Pro Leu Phe Thr Lys
   1265 1270 1275
   Pro Arg Leu Thr Arg Ala Leu
   1280 1285
<210> 3
   <211> 31
   <212> DNA
   <213> Artificial sequence
<220>
   <223> PMT-specific forward primer
<400> 3
   agaggttatg gatccgaaaa caaaacattt t 31
<210> 4
   <211> 20
   <212> DNA
   <213> Artificial sequence
<220>
   <223> oligonucleotide
<400> 4
   atagtagaag aagaattaca 20
<210> 5
   <211> 34
   <212> DNA
   <213> Artificial sequence
<220>
   <223> oligonucleotide
<400> 5
   ctaacataga ggccatggat atgaaaacaa aaca 34
<210> 6
   <211> 34
   <212> DNA
   <213> Artificial sequence
<220>
   <223> oligonucleotide
<400> 6
   acttcgtaca gccatgaatg aaatggctgg aaaa 34
<210> 7
   <211> 20
   <212> DNA
   <213> Artificial sequence.
<220>
   <223> oligonucleotide
<400> 7
   actcaattag aaaaagcgct 20
<210> 8
   <211> 20
   <212> DNA
   <213> Artificial sequence
<220>
   <223> oligonucleotide
<400> 8
   ctactacagt tgctggtatt 20
<210> 9
   <211> 22
   <212> DNA
   <213> Artificial sequence
<220>
   <223> oligonucleotide
<400> 9
   attgttaaga cgtaccagac ga 22
<210> 10
   <211> 33
   <212> DNA
   <213> Artificial sequence
<220>
   <223> oligonucleotide
<400> 10
   ctcttgttaa gctagccttt gtgaaaagag gag 33
<210> 11
   <211> 20
   <212> DNA
   <213> Artificial sequence
<220>
   <223> oligonucleotide
<400> 11
   ttcttccctt aaatcttcag 20
<210> 12
   <211> 32
   <212> DNA
   <213> Artificial sequence
<220>
   <223> oligonucleotide
<400> 12
   tcactggttt ttccagccat ttcattcatg gc 32
<210> 13
   <211> 10
   <212> DNA
   <213> Artificial sequence
<220>
   <223> synthetic random primer
<400> 13
   aagcggcctc